# EUROPEAN PATENT APPLICATION

(11) **EP 1 717 304 A1**
(43) Date of publication of application: **02.11.2006**
(21) Application number: 06252245.3
(22) Date of filing: 26.04.2006
(51) Int. Cl.: C11D 17/04, A61K 8/02, A61Q 19/10

(54) **Cosmetic device comprising nonwoven web**

(30) Priority: 27.04.2005 US 116685
(71) Applicant: JOHNSON & JOHNSON CONSUMER COMPANIES, INC., Skillman, NJ 08558 (US)
(72) Inventor: Eknoian, Michael W., Warren, NJ 07059 (US); Ip, Raymond, Plainsboro, NJ 08536 (US); Poccia, John F., III, Monmouth Beach, NJ 07750 (US); Brennan, Robert Andrew, Jr., Mercerville, NJ 08619 (US)
(74) Representative: James, Anthony Christopher W.P.

(57) **Abstract**

A cosmetic device comprising a solid or semi-solid cosmetic composition and a unitary, nonwoven web is provided. During use of the device by a consumer, the nonwoven web penetrates a surface of the composition, thereby providing a discontinuous surface to the cosmetic device. The cosmetic composition may be a cleansing composition.

## Description

### Background of the Invention

Cosmetic bars have been used over the years to deliver many benefits to the skin. Cosmetic bars containing ingredients such as cocoa butter have been used to moisturize or protect the skin. More conventionally, bars containing soap and moisturizers have been used to cleanse and condition the skin. The bars have several problems associated with them. In particular, one problem is that they are very slippery when wet. The cleansing bars tend to slip out of the hand of the consumer during use in the shower or bath. The consumer then has to bend over or kneel down to pick up the cleansing bar from the floor of the shower.

Additionally, it is somewhat awkward to apply soap with a washcloth or sponge since it involves the use of two separable articles, one being extremely slippery when wet and tending to slide from the user's hands quite easily. Wrapping the washcloth around the soap may be a temporary solution but it is not completely satisfactory. Similarly, making a pouch in the sponge to contain the bar of soap leaves the sponge permanently saturated with the soap and slimy after its initial use. Sewing a bar of soap between two plies of washcloth likewise produces an article that is permanently slimy after use.

Others have tried to extend the life of a cosmetic or cleansing bar that is typically fragile when reduced to a sliver. The sliver will often break or become hand to handle. Solutions to these problems may include the incorporation of hair, sponges, fibers, etc. Examples of such disclosures are described in U.S. Pat. Nos. 681,324; 389,296; 488,393; and 5,221,506.

Skin cleansing compositions having abrasive particles incorporated as scrubbing aids are known in the art. For example, LOOFAH Exfoliating Soap is a commercially available soap bar from Earth Therapeutics. The soap bar has small particles of a chopped up loofah or puff dispersed throughout. Other exfoliating materials are described in U.S. Pat. No. 6,818,603, the contents herein incorporated in its entirety by reference.

The above disclosures fail to provide a cosmetic or cleansing device that demonstrates adequate cleansing, durability, gripability, processibility, lathering and exfoliation properties. Therefore a need still exists for a cosmetic device that provides a pleasant, efficient and easy to handle product for use in the bath or shower. The present invention provides a cosmetic device that resists breakage and, when used with water, controls the amount of solid material going down the drain, thereby minimizing plumbing problems which may result in a clogged pipes.

### Summary of the Invention

The present invention provides a cosmetic device comprising a solid or semi-solid cosmetic composition containing a unitary, nonwoven web, wherein during use the nonwoven web penetrates a surface of the composition, thereby providing a discontinuous surface to the cosmetic device.

The present invention also provides a method of forming a cosmetic device comprising the steps: a) placing a nonwoven web into a mold; b) transferring a flowable cosmetic composition into said mold, such that the composition saturates the nonwoven web; and c) cooling the saturated, nonwoven web into a solid or semi-solid bar that during use comprises a discontinuous surface provided by the nonwoven web.

### Detailed Description of the Invention

As used herein the term "cosmetic" shall include conditioning, moisturizing, cleansing, or any other treatment that is applicable to the skin of the human body. Accordingly, the cosmetic device may be used to deliver any such treatment to the skin.

The cosmetic device comprises a solid or semi-solid cosmetic composition and a unitary, nonwoven web therein.

### Cosmetic Composition

The cosmetic composition may be selected for example from moisturizing compositions, cleansing compositions, or any composition that may provide a benefit to the skin.

In one embodiment, the cosmetic composition is a cleansing composition. Suitable cleansing compositions are solid or semi-solid at room temperature. Examples of useful cleansing compositions include, but are not limited to, fatty acid soaps, including glycerin soaps, synthetic detergents and mixtures thereof. Cleansing compositions are extensively taught in *Soap Technology for the 1990's,* the contents of which are incorporated herein by reference. It is desirable that the cleansing composition be highly flowable when heated, i.e., hot pourable.

In one embodiment of the invention, the cleansing composition comprises glycerin soap. Examples of glycerin soaps useful in the present invention include but are not limited to those disclosed in U.S. Pat. Nos. 4,405,492 and 4,879,063, the disclosures of which are hereby incorporated by reference.

Examples of suitable fatty acid soaps include soaps derived from hydrocarbon chain lengths of from approximately 10 to 22 (including carboxyl carbon) and may be saturated or unsaturated. The soap may be, for example, the sodium salt, potassium salt, ammonium salt, triethanolammonium salt and mixtures thereof.

Suitable synthetic detergents include those known in the art for the desired purpose. Examples of detergents useful for personal cleansing include the isethionates, sarcosinates, and glyceryl ether sulfonates which may be pure chain length variants or those derived from commercial oils such as coconut oil.

Numerous other detergents are appropriate for this invention. These include anionic acyl sarcosinates, methyl acyl taurates, N-acyl glutamates, alkyl sulfosuccinates, alkyl phosphate esters, ethoxylated alkyl phosphate esters, trideceth sulfates, protein condensates, mixtures of ethoxylated alkyl sulfates and alkyl amine oxides, betaines, sultaines and mixtures thereof. Included are the alkyl ether sulfates with 1 to 12 ethoxy groups, especially ammonium and sodium lauryl ether sulfates. Alkyl chains for these other detergents are C₈ -C₂₂, preferably C₁₀ -C₁₈. Alkyl glucosides and methyl glucoside esters are preferred mild nonionics, which may be mixed with other mild anionic or amphoteric surfactants in the compositions of this invention.

In one embodiment, the cleansing composition may comprise the following ingredients:

| | %w/w | |
|---|---|---|
| Propylene Glycol | 32.6 | |
| Glycerin | 12.6 | |
| Sodium Hydroxide Solution (50%) | | 8.6 |
| Stearic Acid | 10.1 | |
| Myristic Acid | 10.1 | |
| Lauric Acid | 8.1 | |
| Water (H2O) | 4.0 | |
| Sodium Lauryl Sulfate | 4.0 | |
| Lauryl Methyl Gluceth-10 Hydroxypropyldimonium Chloride | | 4.0 |
| Cocamidopropyl Betaine | 5.6 | |
| Micro Titamium Dioxide | 0.3 | |
| TOTAL | | 100.0 |

In another embodiment, the cleansing composition comprises these ingredients:

| | %w/w | |
|---|---|---|
| Sodium Cocoyl Isethionate and Stearic Acid | | 59.9 |
| Stearic Acid | 36.7 | |
| Aminomethyl Propanol | 3.4 | |
| TOTAL | | 100.0 |

In yet another embodiment, the cleansing composition comprises a "flexible soap" composition as follows:

| | %w/w | |
|---|---|---|
| Water | 33.59 | |
| Ca-lota Carageenan | | 0.30 |
| Potassium Chloride | 0.70 | |
| Sodium Laureth Sulfate | 24.88 | |
| Kappa Carrageenan | | 1.20 |
| Sodium Cocoyl Isethionate | 1.88 | |
| Glycerin | 36.95 | |
| Phenoxyethanol | 0.50 | |
| TOTAL | | 100.000 |

Optional ingredients conventionally used in cleansing compositions may be incorporated into the cleansing composition of this invention. These ingredients include, but are not limited to, perfumes/fragrances, preservatives, colorants, dyes, anti-caking agents, and personal care ingredients, including, but are not limited to, skin and hair care ingredients.

Examples of suitable personal care ingredients useful in the present invention include but are not limited to safe and effective amounts of: humectants, sunscreen actives, skin soothers, anti-irritants, anti-inflammatories, emollients, conditioning agents, moisturizers, deodorants, anti-perspirants, artificial tanning agents, antimicrobial agents, anti-acne agents, anti-wrinkle agents, anti-skin atrophy agents, skin firming agents, anti-itch agents, anti-fungal agents, topical anesthetics, skin tone evening agents, active natural ingredients, agents for minimizing the appearance or retarding regrowth of unwanted hair, skin texture modifiers, and additional cleansing agents.

Emollients function by their ability to remain on the skin surface or in the stratum corneum to act as lubricants, to reduce flaking, and to improve the skin appearance. Typical emollients include fatty esters, fatty alcohols, mineral oil, polyether siloxane copolymers and the like. Examples of suitable emollients include, but are not limited to, polypropylene glycol ("PPG")-15 stearyl ether, PPG-10 cetyl ether, steareth-10, oleth-8, PPG-4 lauryl ether, vitamin E acetate, PEG-7 glyceryl cocoate, lanolin, and combinations thereof. Vitamin E acetate, PEG-7 glyceryl cocoate and combinations thereof are preferred.

Examples of suitable humectants include polyhydric alcohols. Suitable polyhydric alcohols include, but are not limited to, glycerol (also known as glycerin), polyalkylene glycols, alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof.

Suitable skin soothers include, but are not limited to, panthenol, bisabolol, allantoin, aloe, and combinations thereof.

Suitable conditioning agents include, but are not limited to, dimethicone propyl PG-betaine, dimethicone copolyols, polyquaternium-10, guar, guar derivatives, and combinations thereof. Suitable anti-acne active ingredients include, but are not limited to, salicylic acid, sulfur, lactic acid, glycolic acid, pyruvic acid, urea, resorcinol, N-acetylcysteine, retinoic acid, benzoyl peroxide, octopirox, triclosan, azelaic acid, phenoxyethanol, phenoxypropanol, flavinoids, derivatives thereof, and combinations thereof. Salicylic acid and benzoyl peroxide are preferred.

The optional ingredients may be incorporated directly into the cleansing composition by means known in the art. Alternatively, the optional ingredients may be incorporated into or coated onto the nonwoven web by means known in the art, provided that the optional ingredients sufficiently adhere to the fibers or strands of the nonwoven web until incorporated into the cosmetic device. As used herein "coated" means surface coating and/or at least partially impregnating the fiber, strand or web material. The optional ingredients may be incorporated into or coated onto the fiber, strand or mat material or encapsulated into other components such as a binder used to make the nonwoven web by means known in the art, for example, by treatment with an appropriate solution, suspension or slurry of the ingredient in an appropriate liquid, followed by drying by conventional means. See, for example, U.S. Pat. Nos. 4,335,185; 6,376,072; and 6,420,047, the disclosures of which are hereby incorporated by reference.

In another embodiment, the cosmetic composition is a moisturizing composition. For example, one moisturizing composition comprises the following ingredients:

| | w/w% | |
|---|---|---|
| Isopropyl Palmitate | 50 | |
| Proprietary Polyamide | 15 | |
| Cocamidopropyl Betaine | 5 | |
| Sodium Lauryl Sulfate | | 10 |
| Polyethyleneglycol 400 | 10 | |
| Dimethicone | 10 | |
| TOTAL | | 100 |

### Nonwoven Web

The nonwoven web is substantially contained in the cosmetic composition. That is, a major portion of the nonwoven web, preferably almost all of the nonwoven web, is contained in the cosmetic composition, which is integrated into void spaces of the nonwoven web. In one embodiment, it extends through a major portion of the cosmetic composition, i.e., the nonwoven web extends though at least about 50%, preferably at least about 75%, of the volume of the cosmetic composition.

On use by a consumer, the nonwoven web penetrates a surface of the cosmetic composition, providing a cosmetic device with a rough or discontinuous surface during the lifecycle of the device. The device may or may not comprise a discontinuous surface as manufactured, but on use by a consumer, the nonwoven material is revealed through one or more surfaces of the cleansing composition, thereby providing roughness. By "discontinuous" it is meant that the surface of the cleansing device is not smooth but rather has bumps, irregularities, pits, etc. In another embodiment, the cosmetic device has only one surface that is smooth, i.e., the nonwoven web penetrates all the other surfaces. Such a discontinuous surface provides gripping properties to the cosmetic device. The user is less likely to drop the cosmetic device, thereby providing a more pleasant experience for the user.

Such a discontinuous surface is also advantageous when the cosmetic device comprises a cleansing composition. When the device is used for cleansing, one or more discontinuous surfaces may be used to exfoliate the outer layer of the skin. This typically involves removing the dead cells from the stratum corneum. The ability to rub the skin with the nonwoven web may, for example, replace the need for a washcloth, loofa, puff, or other implement typically used by consumers to clean and stimulate their skin in the bath/shower.

The nonwoven web is a unitary structure. It is relatively stable and not capable of being readily separated except by force. During use, the nonwoven web does not substantially separate or slough off. It remains substantially intact under normal use conditions. When the cosmetic device is "used up," the device will be substantially free of cosmetic composition and will be primarily a mat of nonwoven material, which can easily be discarded.

The nonwoven web may be made of any synthetic or natural nonwoven material. It may comprise individual elements such as fibers, strands, filaments, etc., integrated into a web. It may be in the form of a single layer or multiple layers. In the latter embodiment, the layers are stacked one on top of another and temporarily joined together, for instance, by a soluble material. For example, two webs are joined by first spraying a soluble material to the upper surface of the first web. The second web is then secured to the top of the first. This dual layer nonwoven web is then used to form the cosmetic device. During use, especially as a cleansing device, the cosmetic composition will be substantially worn away. The user can then remove one of the layers, discard it and continue using the device.

The nonwoven web may be, for example, made of airlaid, entangled including hydroentangled, thermally bonded, or latex bonded material. Highloft nonwovens are low density, and thick or bulky. They are characterized by a high ratio of thicknesss to weight per unit area. Highloft battings have no more than 10% solids by volume and are greater than 3mm (0.013") in thickness.

Fibers used to make the nonwoven web may be any of several water-insoluble synthetic fibers, ranging from acetate rayon and cellulose (which are relatively supple when wetted with water) to nylon, polyester and isotactic polypropylene (which are relatively firm). Fibers such as nylon and polyester may be oriented to provide further resilience and strength. The type, diameter and length of the fibers may vary according the desired use. For example, a relatively thick resilient fiber may be used in a device for washing the hands while thin and more supple fiber may be used in a device used as a bath soap.

The synthetic fibers may be comprised of polyamides, such as poly(hexamethylene adipamide), polycaproamide and/or copolymers thereof; polyesters, such as poly(ethylene terephthalate); poly(hexahydro-p-xylylene terephthalate), and/or copolymers; polyolefins, such as polypropylene and polyethylene; polyurethanes, polycarbonates, polyacetals, polyacrylics, vinyl polymers, vinylidene polymers, nylon, and the like.

The nonwoven web preferably has appropriate loft and dimensional stability. For example, in forming a cosmetic device comprising a cleansing composition that is 1 inch thick, the nonwoven web must ultimately retain enough height after the process to extend through a major portion of the cleansing composition.

In one embodiment, the nonwoven web is made of high loft airlaid polyester fibers bound with acrylic polymer. in general, conventional binders known in the art may be used, for example ethylene vinyl acetates, vinyl acetates, acrylics, styrene vinyl acetates, startches, and poly vinyl alcohols.

In another embodiment, the nonwoven web has a force of greater than 28 N. This advantageously provides nonwoven web less susceptible to pilling and breakage without compromising lathering, skin feel, exfoliation, and grip-ability. A higher force/density ratio also aides in manufacturing and processing of the cosmetic device.

For purposes of this invention, force of a nonwoven is calculated using an Instron Universal Tester Model 1122 with MTS ReNew®, based on ASTM Method D-882. Width is set at 52 mm (2 inches). Crosshead is set at 100 mm per minute. Gage length is set at 50 mm. Break sensitivity is set at 95%. Peak load in N per square inch width is reported.

The test is performed by cutting nonwoven web samples 2 inches wide (CD) on a cutting board. Each sample is cut 7 inches long (MD). Pneumatically operated rubber-faced clamps clamp the sample securely such that a gage length of 50 mm of web is between the clamps. The test stretches the sample in the machine direction to determine the maximum force required to tear it asunder, n=3.

In one embodiment, the nonwoven web comprises hollow fibers as taught in U.S. Pat. Nos. 3,558,420 and 5,937,874, the entirety of both are hereby incorporated by reference. The hollow fibers may be utilized to contain optional ingredients as described above. In this embodiment, the optional ingredients may be drawn into the fiber by capillary action or through the use of vacuum. The fibers may have walls that fracture upon use of the cosmetic device, thereby releasing the optional ingredient(s).

The cosmetic device of the present invention provides a solution to common problems associated with many types of bars including bath soaps. It provides an efficient tool for exfoliation and can improve the handling of a bar of soap. Additionally, the present invention can avoid clogging of drains by utilizing a nonwoven web that is stable and does not substantially break up into smaller portions that might flow into a drain in the shower, sink or bath.

The cosmetic device may be made by any of the conventional methods known in the art. These methods include but are not limited to hot pour and extrusion methods, the particulars of which are known by those skilled in the soap art.

In one embodiment, the cosmetic device may be prepared by heating a cosmetic composition to a temperature at which it flows, i.e., above its melting point (typically about 70° C. to about 130° C.). A nonwoven web such as a high loft material is placed into a mold, such as one made of plastic or rubber. The composition is added into the mold with the high-loft material. The composition saturates the nonwoven web, and the nonwoven web extends through a major portion of the composition. The composition is allowed to cool and harden into a solid or semi-solid state. The device is de-molded and optionally cut into appropriate shapes and sizes.

Optional ingredients like perfume, skin care ingredients, and colorants may be added.

### Examples

### Example 1

792 grams of commercially available glycerin soap bars were heated to approximately 70° C with moderate stirring until the bars completely melted. While the bars were melting, 8 grams of a 75%PET/25% acrylic polymer airlaid high-loft material was placed into a rectangular mold approximately 3"x 2"x 8". The molten soap was poured into the mold with the nonwoven web and allowed to cool. The soap was demolded after solidifying. The soap was cut into 8 individual rectangular bars 3" x 2" x 1" into cosmetic devices according to the invention.

### Example 2

Cosmetic devices according to the invention were made as follows.

27.2 grams of aminomethyl propanol was introduced to a glass container. Then, 293.4 grams of stearic acid was added and the mixture was heated to 75-95° C with stirring at 200-300 rpm. Once the ingredients were well-mixed, 479.4 grams of BASF Jordapon Cl 65 (a blend of sodium cocoyl isethionate and stearic acid) were added to the glass container while the temperature was kept in the same range as before. The ingredients were then mixed for approximately another 10 minutes. While the mixing was taking place, pieces of 75%PET/25% acrylic polymer high-loft material (previously cut to have a similar shape as a mold and be approximately 0.5 grams in weight) were placed into each of 6 individual soap molds. When the mixing of the ingredients in the glass container was completed, about 100-120 grams of the mixture was poured into each mold with the high loft material and allowed to cool. The resulting solid materials were then demolded.

### Example 3

About 450 grams of a pre-made glycerin soap base was melted. While the soap base was being melted, a piece of high-loft material made of 6-denier PET fibers and Vinamul binder Elite 22 with a weight of about 0.30 grams was placed in each of 5 soap molds. The molten soap base was then introduced into each of the 5 molds until the combined weight of the soap base and high-loft in each mold equaled approximately 30 grams. Each mixture was allowed to cool and solidify into cosmetic devices according to the invention.

As a comparison, five soap bars of 30 grams each were prepared in identical molds except no high-loft materials were included in the bars. The comparative bars were allowed to cool and solidify.

The gripability of the 10 bars was tested as follows. The 10 bars were preconditioned prior to testing by wetting each in warm water, then rubbing each in the palm with 10 circular motions. The wetting and rubbing steps were repeated two additional times.

A 14-degree slanted surface was created using a lab jack and a flat and smooth tray. A transparent film was placed on the board to allow for the tracking of each bar's motion as the bar travels down the surface). A starting line was marked on the tray.

Each of the 10 pre-conditioned bars were again wetted in warm water, rubbed in the palm with 10 circular motions and then released from the premarked starting point on the slanted surface. Each bar was allowed to travel down the surface for 20 seconds. The bar was then stopped and the distance traveled by the bar was recorded (the finishing point was defined as the point of the bar which was the farthest down the slope). The same procedure was followed for all 10 bars and each bar was tested twice. An average distance traveled in 20 seconds was then calculated for each bar.

The comparative bars consistently traveled down the slanted surface by a longer distance than the devices of the invention. On average, the comparative bars slipped down the slanted surface 5.09 inches, whereas the devices according to the invention traveled minimal distances averaging 0.04 inches. The difference was statistically significant (p < 0.02). In other words, the comparative bars tended to be more slippery than those made according to the invention.

### Example 4

The following nonwoven webs were tested for force according to the procedure described above.

| | | | | | Peak Force per 2" | |
|---|---|---|---|---|---|---|
| | | | | | Width *(units: Newtons)* | |
| Supplier | Fiber | Binder | Approximate Weight, osf | Approximate Thickness, inches | *Mean* | *Standard Deviation* |
| | | | | | | |
| Carlee Corporation (Rockleigh, NJ) | 15 dpf polyester | Vinamul Binder NACrylic X4484 | 0.28 | 1.0 | 27.08 | 3.30 |
| Carlee Corporation (Rockleigh, NJ) | 15 dpf polyester | Vinamul Binder AR-7 | 0.28 | 1.0 | 55.75 | 2.89 |
| Hollinee LLC/ Ahlstrom Corporation (Groesbeck, TX) | 6 dpf polyester | Vinamul Binder NACrylic X4484 | 0.37 | 0.9 | 41.49 | 3.56 |
| Hollinee LLC/ Ahlstrom Corporation (Groesbeck, TX) | 6 dpf polyester | Vinamul Binder Elite 22 | 0.39 | 1.0 | 63.39 | 6.84 |
| Hollinee LLC/ Ahlstrom Corporation (Groesbeck, TX) | 3 dpf polyester | Vinamul Binder Elite 22 | 0.33 | 0.6 | 49.09 | 3.92 |

## Claims

1. A cosmetic device comprising a solid or semi-solid cosmetic composition containing a unitary, nonwoven web, wherein during use the nonwoven web penetrates a surface of the composition, thereby providing a discontinuous surface to the cosmetic device.

2. The cosmetic device of claim 1, wherein said nonwoven web extends through a major portion of the composition.

3. The cosmetic device of claim 2, wherein said nonwoven web extends through at least about 75% of the volume of the composition.

4. The cosmetic device of claim 1, wherein the cosmetic composition is a cleansing composition.

5. The cosmetic device of claim 1, wherein the cosmetic composition is a moisturizing composition.

6. The cosmetic device of claim 1, wherein the discontinuous surface of the cosmetic device is capable of exfoliating the stratum corneum.

7. The cosmetic device of claim 1, wherein the cosmetic composition is hot pourable.

8. The cosmetic device of claim 1, wherein the discontinuous surface provides gripping properties.

9. The cosmetic device of claim 1, wherein the nonwoven web has a force of greater than 28 N.

10. The cosmetic device of claim 1, wherein the nonwoven web comprises fiber selected from the group consisting of absorbent fiber, cellulosic fiber, and polyester fiber.

11. The cosmetic device of claim 1, wherein the nonwoven material comprises multiple layers.

12. The cosmetic device of claim 11, wherein the layers are joined by a soluble material.

13. A method of forming a cosmetic device comprising the steps:
a) placing a nonwoven web into a mold;
b) transferring a flowable cosmetic composition into said mold, such that the composition saturates the nonwoven web; and
c) cooling the saturated, nonwoven web into a solid or semi-solid bar that during use comprises a discontinuous surface provided by the nonwoven web.

14. The method of claim 13, wherein said nonwoven web extends through a major portion of the composition.

15. The method of claim 14, wherein said nonwoven web extends through at least about 75% of the volume of the composition.

16. The method of claim 13, wherein the cosmetic composition is a cleansing composition.

17. The method of claim 13, wherein the cosmetic composition is a moisturizing composition.

18. The method of claim 13, wherein the cosmetic composition is hot pourable.

19. The method of claim 13, wherein the nonwoven web has a force of greater than 28 N.

20. The method of claim 13, wherein the nonwoven web comprises fiber selected from the group consisting of absorbent fiber, cellulosic fiber, and polyester fiber.
